# EUROPEAN PATENT SPECIFICATION

(11) **EP 3 065 548 B1**
(45) Date of publication and mention of the grant of the patent: **21.08.2019**
(21) Application number: 14849792.8
(22) Date of filing: 29.09.2014
(51) Int. Cl.: A01N 43/00, A61K 31/33, A61K 31/41, A61K 31/425, A61K 31/38, A01N 37/18, A61K 31/16, A61P 11/06, A61P 11/00, A61P 1/16, A61P 1/00, A61P 9/00, A61P 13/12, A61P 17/00, A61P 19/00, A61P 43/00

(54) **FIBROSIS INHIBITING COMPOUNDS FOR USE IN THE PREVENTION OR TREATMENT OF FIBROSING DISEASES**
FIBROSEHEMMENDE VERBINDUNGEN ZUR VERWENDUNG FÜR DIE VORBEUGUNG ODER BEHANDLUNG GRANULOMATÖSER ERKRANKUNGEN
COMPOSÉS INHIBITEURS DE FIBROSE POUR PRÉVENIR OU TRAITER LES MALADIES FIBROTIQUES

(30) Priority: 30.09.2013 US 201361884384 P
(43) Date of publication of application: 14.09.2016
(73) Proprietor: THE TEXAS A&M UNIVERSITY SYSTEM, College Station, Texas 77843-3369 (US)
(72) Inventor: GOMER, Richard, H., College Station, TX 77845 (US); COX, Nehemiah, College Station, TX 77840 (US)
(74) Representative: Müller-Boré & Partner Patentanwälte PartG mbB
(86) International application number: PCT/US2014/058040
(87) International publication number: WO 2015/048641

(56) References cited:
- WO-A1-2013/095973
- US-A1- 2009 263 378
- VERED MOLINA ET AL: "Immunomodulation of experimental pulmonary fibrosis by intravenous immunoglobulin (IVIG)", AUTOIMMUNITY, vol. 39, no. 8, 1 January 2006 (2006-01-01), pages 711-717, XP055336274, GB ISSN: 0891-6934, DOI: 10.1080/08916930601061272
- TONG ZHOU ET AL: "Effects of DC-SIGN expression on renal tubulointerstitial fibrosis in nephritis", FRONTIERS IN BIOSCIENCE, vol. 14, no. 8, 1 January 2009 (2009-01-01), pages 2935-2943, XP055348619, US ISSN: 1093-9946, DOI: 10.2741/3424
- M. JACK BORROK ET AL: "Non-carbohydrate Inhibitors of the Lectin DC-SIGN", JOURNAL OF THE AMERICAN CHEMICAL SOCIETY, vol. 129, no. 42, 1 October 2007 (2007-10-01), pages 12780-12785, XP055336275, US ISSN: 0002-7863, DOI: 10.1021/ja072944v
- INESSA SCHWAB ET AL: "Intravenous immunoglobulin therapy: how does IgG modulate the immune system?", THE JOURNAL OF IMMUNOLOGY, vol. 13, no. 3, 1 March 2013 (2013-03-01), pages 176-189, XP055234901, GB ISSN: 1474-1733, DOI: 10.1038/nri3401
- MOLINA ET AL.: 'Immunomodulation of experimental pulmonary fibrosis by intravenous immunoglobulin (IVIG).' AUTOIMMUNITY. vol. 39, no. 8, 2006, pages 711 - 7, XP055336274
- SCHWAB ET AL.: 'Intravenous immunoglobulin therapy: how does IgG modulate the immune system;' NAT REV IMMUNOL. vol. 13, no. 3, March 2013, pages 176 - 89, XP055234901
- BORROK ET AL.: 'Non-.carbohydrate Inhibitors of the Lectin DC-.SIGN.' JOURNAL OF THE AMERICAN CHEMICAL SOCIETY vol. 129, no. 42, 2007, pages 12780 - 12785, XP055336275

## Description

The present invention relates to a CD209-binding compound for use in a method of suppressing fibrocyte or profibrotic macrophage formation, wherein the compound is administered to monocytes in a target location in a lung of a mammal that has fibrosis or a fibrosing disease affecting the lung.

### Fibrosing Diseases

Fibrosis is an important part of the natural wound healing process. However, when fibrosis is not regulated properly by the body, it results in scar tissue being formed in places where it does not belong, often causing serious injury and pain to patients and, in many cases, even endangering their lives. Common fibrosing diseases include scleroderma (a skin and internal organ fibrosis), pulmonary fibrosis, cirrhosis of the liver, renal fibrosis, and cardiac fibrosis. In each of these fibrosing diseases, scar tissue is formed in the affected organs, impeding their function, often eventually to the point where the patient dies from organ failure. Thousands of patients are affected by these and other fibrosing diseases each year in the US alone. Despite the high prevalence of fibrosing diseases, there are no effective FDA-approved therapies to dates.

### How a Fibrosing Disease Develops

Fibrosis is largely controlled by the immune system and a variety of immune system cells participate in the process of scar tissue formation and fibrosis. At a normal wound site, such as a cut, monocytes, a circulating immune cell, leave the blood, enter the tissue near the lesion, and differentiate into profibrotic macrophages and fibrocytes, both of which contribute to fibrosis and wound healing. In particular, fibrocytes express components of scar tissue, such as collagen. After adequate healing has taken place, production of wound healing signals and materials by fibrocytes and profibrotic macrophages ceases.

In patients with a fibrosing disease, this process can go wrong in a variety of ways. Fibrotic lesions may appear inappropriately. The movement of monocytes into a tissue may occur where there is no wound. Too many monocytes may enter the wound. Fibrocytes may not cease production of wound healing materials at the appropriate time. Profibrotic macrophages may release signaling molecules to initiate scar tissue formation where there is no damage. Other problems may also occur, however, the effects of all of these and potentially other inappropriate responses may be prevented or diminished by decreasing the ability of monocytes to form fibrocytes, by shifting macrophages towards an immuno-regulatory M2 phenotype, or inhibiting the formation of profibrotic macrophages.

### Serum Amyloid P (SAP) and C - reactive protein (CRP)

Serum amyloid P (SAP) is a 125 kDa, pentameric, pentraxin protein that has recently been identified as an anti-fibrotic agent able to inhibit the formation of fibrocytes from monocytes. SAP also inhibits the formation of profibrotic macrophages in mouse models of pulmonary fibrosis. In mouse models of renal fibrosis, SAP promotes the formation of immuno-regulatory M2 macrophages that can suppress the immune system and thus fibrosis via the secretion of the signaling protein interleukin 10. SAP has been proven to alleviate fibrosis in animal models of pulmonary fibrosis, kidney fibrosis and cardiac fibrosis. SAP mediates its effect partly through a group of extracellular receptors known as Fc gamma receptors, which are better known for binding IgG antibodies. SAP may also bind unknown receptors on monocytes.

In contrast to SAP, the very similar C-reactive protein (CRP), which shares a 51% sequence identity with SAP, has no appreciable effect on inhibiting fibrocyte differentiation. In addition, CRP binds macrophages to form pro-inflammatory M1 macrophages that could worsen fibrosis.

### Glycosylation

The regulation of the immune system by proteins often depends on the glycosylation of those proteins. Glycosylation refers to the attachment of carbohydrate molecules to proteins. Glycosylation is typically triggered by certain amino acids on the proteins. Cells can create diverse patterns of protein glycosylation to convey different messages. This phenomenon can be observed in antibodies (gamma globulins). Antibodies with fully processed N-linked glycans terminating in sialic acid bind macrophages and dendritic cells to up-regulate Interleukin 33 in the immune system. This ultimately leads to decreased sensitivity of macrophages to proinflammatory signals. In contrast, antibodies lacking terminal sialic acid bind immune cells to initiate an immune response.

Glycosylation, in addition to providing a plethora of conveyable messages, also acts as an identification key because the glycosylation patterns used by cells can be species specific. This uniqueness allows the immune system to recognize foreign and sometimes dangerous proteins or pathogens and to initiate an immune response.

### Glycosylation Receptors

Many cells in the human body have receptors on their surfaces that detect glycosylated proteins. These receptors are called lectin receptors and are important in receiving messages conveyed by glycosylation and in recognizing glycosylation patterns.

CD209 (also known as Dendritic Cell-Specific Intercellular adhesion molecule-3-Grabbing Non-integrin (DC-SIGN)) is a lectin receptor that uses calcium as a co-factor to bind glycosylated proteins. CD209 can be found on the surface of monocytes, macrophages, and dendritic cells and binds mannose carbohydrates on pathogens and partially glycosylated proteins to initiate an immune response. This receptor also binds to fucose carbohydrates on the surface of fully processed glycosylated proteins to prevent the immune system from attacking the host.

Recently, CD209 was also recognized as a receptor for immuno-suppressive gamma globulins containing α(2,6)-glycosidic linkages of sialic acid as the terminal carbohydrate on their surfaces. This specific type of glycosylation binds CD209 to up-regulate the secretion of the cytokine, Interleukin 33. The end result of this up-regulation is decreased sensitivity of macrophages to proinflammatory molecules that signal through Fc gamma receptors.

In this context, WO 2013/095973 A1 describes DC-SIGN binding peptides and proteins and uses thereof in modulating immune responses and treating immune disorders. The present invention relates to the following items:
1. A CD209-binding compound for use in a method of suppressing fibrocyte or profibrotic macrophage formation, wherein the compound is Compound 1 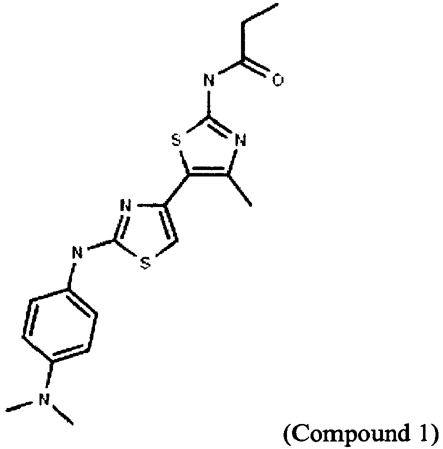 and is administered to monocytes in a target location in a lung of a mammal that has fibrosis or a fibrosing disease affecting the lung.
2. The compound for use of Item 1, wherein the compound is administered by a mode selected from the group consisting of: topically, orally, by injection, by intravenous injection, by inhalation, continuous release by depot or pump, or any combinations thereof.
3. The compound for use of Item 1, wherein the compound is administered in an amount and for a time until the target location in the mammal contains the compound in an amount of at least 3 pM.

Described herein is a method of inhibiting fibrocyte or profibrotic macrophage formation by administering a CD209-binding compound to monocytes in a target location in an amount and for a time sufficient to suppress formation of fibrocytes or profibrotic macrophages from the monocytes. Further, described herein are compounds and pharmaceutical formulations for use in such methods. Furthermore, described herein is a method of treating a fibrosing disease by administering a CD209-binding compound or antibody to the patient in an amount and for a time sufficient to suppress formation of fibrocytes or profibrotic macrophages from the monocytes.

Elements of the method described in the preceding paragraph may also be used in combination with any of the following elements, which may also be combined with one another as appropriate. A. The mammal may be a human. B. The compound may be administered by a mode selected from the group consisting of: topically, oral, by injection, by inhalation, continuous release by depot or pump, or any combinations thereof. C. The compound may be administered by intravenous injection. D. The mammal may have a fibrosing disease. E. The mammal may have scleroderma. F. The mammal may have pulmonary fibrosis. G. The mammal may have asthma. H. The mammal may havefibrosis or a fibrosing disease affecting the liver, lung, heart, pericardium, eye, skin, mouth, esophagus, pancreas, gastrointestinal tract, brain, breast, bone, bone marrow, or genitourinary system, or resulting from radiation. I. Administering may include administering until the target location in the mammal contains the compound in an amount of at least 3 pM. J. Administering may include administering until the target location in the mammal contains the compound in an amount of at least 300 pM. K. Administering may include administering until the target location in the mammal contains the compound in an amount of at least 2 nM. L. The compound may include Compound 1. M. The compound may include a compound of formula I in which R₁ includes an aryl group, an alkyl group, a substituent with both aryl and alkyl components, or H. N. The compound may include Compound 2. O. The compound may include Compound 3. P. The compound may include a compound of formula II, wherein R₁ includes an aryl group, an alkyl group, a substituent with both aryl and alkyl components, or H, and R₂ and R₃ both include a monovalent halide, an aryl, an alkyl, a substituent with both aryl or alkyl components, or H. Q. The compound may include a compound of formula III, wherein R₁, R₂, and R₃ each include an alkyl group, an aryl group, a substituent with both aryl and alkyl components, or H. R. The compound of Q, wherein R₁ and R₃ both include an aryl group. S. The compound includes a compound of formula IV or its derivatives. T. The compound includes a compound of formula V and/or its derivatives. U. The compound includes a compound of formula VI, wherein R includes an aryl group, an alkyl group, a substituent with both aryl and alkyl components, or H. V. The compound includes a compound of formula VII, wherein R comprises an aryl group, an alkyl group, a substituent with both aryl and alkyl components, or H. W. The compound includes Compound 24. X. The compound includes Compound 25. Y. The compound includes Compound 26. Z. The compound includes Compound 27. AA. The antibody includes anti-human CD209 anti bodies. BB. The anti-human CD209 antibody is selected from the group consisting of clone 9E9A8, clone H-200, clone DCN4. clone eB-h209, and clone ERP5588. CC. Antibodies may include monoclonal antibodies, polyclonal antibodies, antibody fragments, human antibodies, humanized antibodies, chimeric antibodies, bispecific antibodies, recombinant antibodies, IgG1 antobodies, and IgG2 antibodies. LL. Antibodies may include antibodies that bind to the same epitopes as clone 9E9A8, clone H-200, clone DCN4, clone eB-h209, or clone ERP5588. MM. Antibodies may be administered at a concentration of 10 micrograms/ml or less. NN. Antibodies may be administered at a concentration of 1 micrograms/ml or less. OO. Antibodies may be administered at a concentration of 0.1 micrograms/ml or less. PP. Antibodies may be administered at a concentration of between 0.1 micrograms/ml and 10 micrograms/ml. QQ. Antibodies may be administered at a concentration of between 0.1 micrograms/ml and 10 micrograms/ml.

Further described herein is a screening method including screening a compound or antibody to detect whether it binds CD209, then selecting a compound or antibody able to bind CD209 and further screening the compound or antibody to determine if it inhibits fibrocyte or profibrotic macrophage formation. A compound or antibody thus selected may have any combination of elements of any of the two preceding paragraphs. Screening a compound or antibody to detect whether it bind CD209 may include high-throughput screening.

A more complete understanding of embodiments of the present disclosure and advantages thereof may be acquired by referring to the following description taken in conjunction with the accompanying drawings.

The figures show:
FIGURE 1A shows an SDS-PAGE protein gel of SAP, desialylated SAP (SAP NA), CRP, and mutated-glycosylated CRP (CRP A32N) stained with silver stain to detect protein.
FIGURE 1B shows a western blot of the samples shown in FIGURE 1A, stained with biotinylated Sambucus Nigra lectin to detect α (2,6)-glycosidic linkages of sialic acid.
FIGURE 2 presents the effects of CRP, mutated-glycosylated CRP (CRP A32N), and SAP on fibrocyte count after exposure of peripheral blood mononuclear cells (PBMCs) to the relevant protein. Values are mean +/- standard error of mean (SEM) (n=3) of fibrocyte counts normalized to a no-protein control. Lines represent sigmoidal dose-response curves fit to the data. * indicates significant difference between CRP A32N and CRP with p < 0.05 (t-test).
FIGURE 3 presents the effect of normal wild-type SAP (SAP) and enzymatically desialylated SAP (SAP NA) on fibrocyte differentiation from monocytes. Values are mean +/- standard error of mean (SEM) (n=3) of fibrocyte counts normalized to a no-protein control. * indicates significant difference compared to SAP with p < 0.05 (t-test).
FIGURE 4 presents the effect of CRP, mutated-glycosylated CRP (CRP A32N), SAP, and enzymatically desialylated SAP (SAP NA) on macrophage polarization to either the M1 or M2 phenotype as measured by the M1 marker ICAM-1 and the M2 marker CD163. Values are mean +/- standard error of mean (SEM) (n=3). * indicates significant difference compared to the control with p < 0.05 (t-test).
FIGURE 5 presents the effect of anti-human CD209 antibodies on fibrocyte counts after exposure of PBMCs to the antibody. Human PBMCs were treated with the indicated concentrations of the antibodies. Subtype matched IgG was used as the negative control for each tested antibody. Values are mean +/- SEM (n=3) of counts normalized to the no antibody-control. * indicates p<0.05 by t-test.
FIGURE 6 presents the effects of Compound 1 and Compound 2 on fibrocyte count after exposure of PBMCs to the small molecules. Values are mean +/- SEM (n=4) of fibrocytes. Counts are normalized to the no-compound control. Lines are sigmoidal dose-response curves fit to the data.
FIGURE 7 presents the effects of Compound 3 on fibrocyte count after exposure of PBMCs to the small molecule. Values are mean +/- SEM (n=3). Counts are normalized to the no-compound control. The line is a linear fit to the data.
FIGURE 8 presents the effects of Compounds 1, 2 and 3 on cell viability as measured using Alamar Blue. Values are mean +/- SEM (n=3) of fluorescence intensity. Values are normalized to the non-compound control.
FIGURE 9A illustrates the effect of compound 1 on inflammatory macrophages (CD11b) and alveolar macrophages (CD11c) in the bronchoalveolar lavage fluid from the lungs of C57BL/6 mice. Rat IgG1 indicates staining with a rat isotype control antibody. Values are mean ± SEM, n=4. * represents p<0.05 and ** represents p<0.01 by 1-way ANOVA, Holm-Bonferroni test.
FIGURE 9B illustrates the effect of compound 1 on inflammatory macrophages (CD11b) and alveolar macrophages (CD11c) in the lung sections of C57BL/6 mice. Rat IgG1 indicates staining with a rat isotype control antibody. Values are mean ± SEM, n=4. * represents p<0.05, ** represents p<0.01 and *** represents p<0.001 by 1-way ANOVA, Holm-Bonferroni test.
FIGURE 10A shows mouse lung sections stained for collagen using picrosirius red staining. Bar is 250 microns.
FIGURE 10B shows the percent collagen staining in mouse lung sections as determined by picrosirius red staining. Values are mean ± SEM, n=4. ** represents p<0.01 and *** represents p<0.001 by 1-way ANOVA, Holm-Bonferroni test.

The present disclosure relates to compounds able to inhibit fibrosis by inhibiting the formation of fibrocytes or profibrotic macrophages from monocytes. In particular, it relates to compounds able to bind to CD209 on the surface of monocytes or monocyte-derived cells. The present disclosure further relates to pharmaceutical formulations of such compounds suitable for the invention or treatment of fibrosing diseases and to method of preventing or treating fibrosing diseases using such compounds.

### CD 209-Binding Compounds

CD209 is an extracellular protein found on the surface of monocytes and other immune system cells. CD209 binds to α(2,6)-glycoside linkages of sialic acids on gamma globulins to up-regulate the secretion of the cytokine Interleukin 33. The end result of this up-regulation is decreased sensitivity of macrophages to proinflammatory molecules that signal through Fc gamma receptors. The SAP protein has this type of glycosylation, while CRP does not. This difference in glycosylation accounts for the different effects of these two proteins on CD209. Accordingly, compounds able to bind to CD209 in the same manner as α(2,6)-glycoside linkages of sialic acid or to otherwise bind to it in such a way as to alter monocyte function may inhibit the formation of fibrocytes or profibrotic macrophages from monocytes, for example by either preventing or decreasing such formation, such as by inhibiting differentiation of monocytes into fibrocytes or by shifting macrophage polarization from the proinflammatory M1 phenotype or the profibrotic M2 phenotype to the immuno-regulatory M2 phenotype. Such compounds may be used to prevent or treat fibrosing diseases.

Compounds able to bind CD209 may include both small molecules and biological molecules, such as antibodies.

In one embodiment, small molecule compounds may be based on mannose, mannose-containing oligosaccharides, or mannose mimetics. Mannose is a natural ligand for CD209.

In another embodiment, small molecule or biological molecule compounds may contain a mannose or mannose-like sugar epitope on a peptide, dendrimeric scaffold, or a polymer. Embodiments containing multiple epitopes may be capable of multivalent binding. Multivalent binding may lead to greater inhibition of fibrocyte formation.

In another embodiment small molecule compounds may be based on fucose, fucose-containing oligosaccharides, or fucose mimics. Fucose is a natural ligand for CD209.

In another embodiment, small molecule or biological molecule compounds may contain a fucose or fucose-like sugar epitope on a peptide, dendrimeric scaffold, or a polymer. Embodiments containing multiple epitopes may be capable of multivalent binding. Multivalent binding may lead to greater inhibition of fibrocyte formation.

In another embodiment, the compound may be a non-carbohydrate small molecule inhibitor. Such inhibitors may bind CD209 much more strongly than carbohydrate-based small molecules. In a more specific embodiment, such a compound may contain at least one amide carbonyl group, which may interact with a cation, particularly a divalent cation, such as Ca²⁺, when binding to CD209. In another more specific embodiment, such a compound may contain at least one aromatic group. In a further specific embodiment, the compound may contain both at least one amide carbonyl group and at least one aromatic group.

According to the present invention, the compound has the chemical formula: 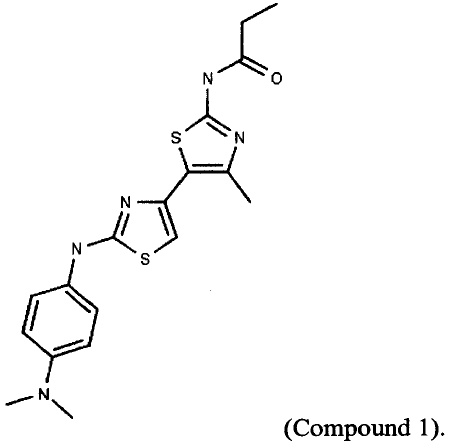 Compound 1 has an affinity for CD209 in which K_{d} = 1.6 +/- 0.5 µM.

Further, described herein is a compound having the general chemical formula: 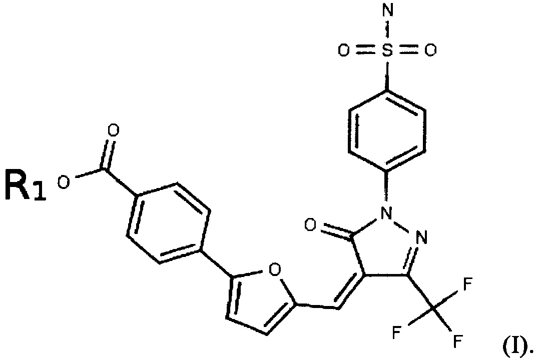 In more specific embodiments, R₁ may be a propyl (Pr) group (Compound 2). Compound 2 has an affinity for CD209 in which K_{d}=6.2 +/- 3.0 µM. In another specific embodiment, R₁ may be a methyl (Me) group (Compound 3). Compound 3 has an affinity for CD209 in which K_{d}=32 +/- 18 µM. In still other embodiments, R₁ may be an aryl group, an alkyl group, a substituent with both aryl and alkyl components, or H.

Further, described herein is a compound having the general chemical formula: 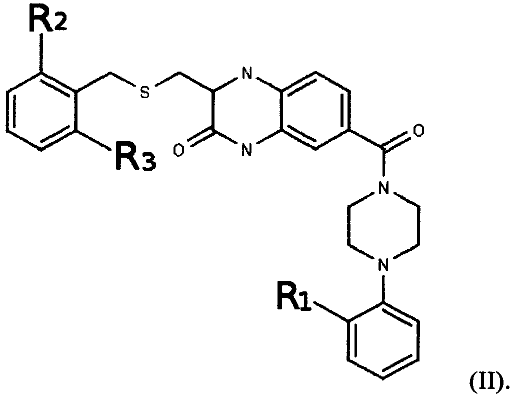 In more specific embodiments, R₁ may be an aryl group, an alkyl group, a substituent with both aryl and alkyl components, or H. R₂ and R₃ may be a monovalent halide, an aryl, an alkyl, a substituent with both aryl or alkyl components or H.

Further, described herein is a compound having the general chemical formula: 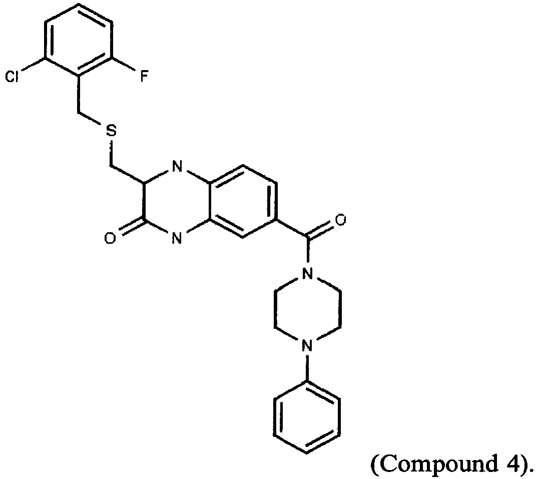 Compound 4 has an affinity for CD209 in which K_{d}=7.3 +/- 3.5 µM.

Further, described herein is a compound having the general chemical formula: 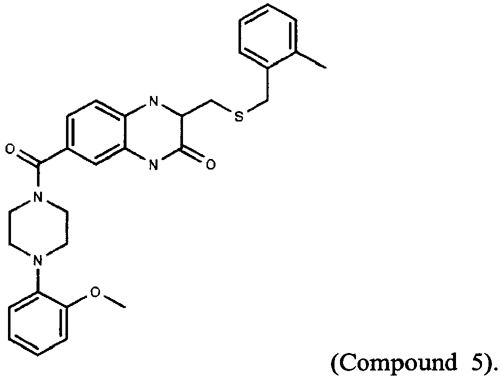 Compound 5 has an affinity for CD209 in which K_{d}=2.0 +/- 1.0 µM.

Further, described herein is a compound having the general chemical formula: 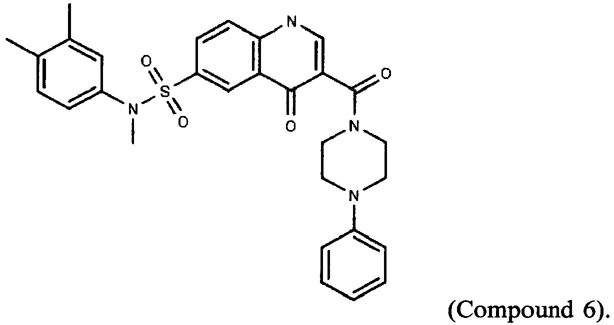 Compound 6 has an affinity for CD209 in which K_{d}=6.1+/- 3.0 µM.

Further, described herein is a compound having the general chemical formula: 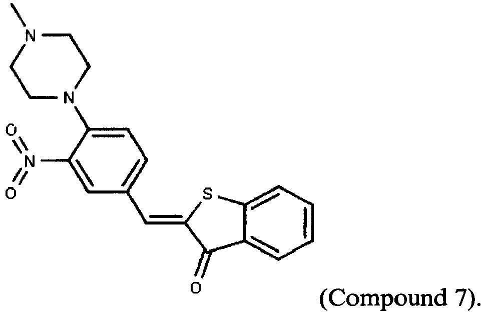 Compound 7 has an affinity for CD209 in which Kd=10.0+/- 9.0 µM.

Further, described herein is a compound having the general chemical formula: 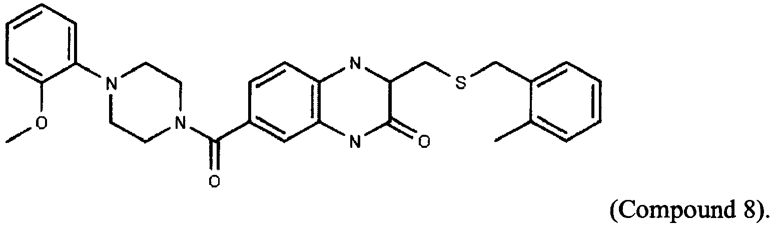 Compound 8 has an affinity for CD209 in which K_{d}=7.0 µM.

Further, described herein is a compound having the general chemical formula: 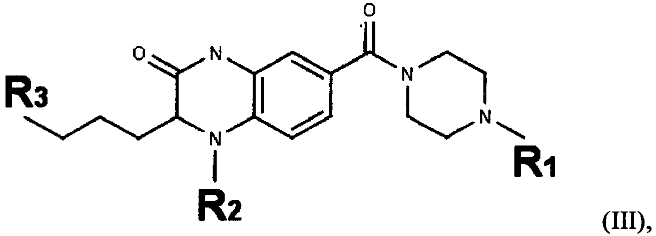 in which R₁, R₂ and R₃ are presented in TABLE 1 for various compounds.

**Table 1 - R groups for Formula III**

| **Compound** | **R₁** | **R₂** | **R₃** | **IC₅₀ (µM)*** |
|---|---|---|---|---|
| 9 | propyl (Pr) | ethyl (Et) | ethyl (Et) | 370 +/- 70.0 |
| 10 | ethyl (Et) | methyl (Me) | ethyl (Et) | 360 +/- 69.2 |
| 11 | methyl (Me) | methyl (Me) | methyl (Me) | 329 +/- 65.8 |
| 12 | methyl (Me) | H | methyl (Me) | 313 +/- 47.7 |
| 13 | tert-Butoxycarbonyl (Boc) | ethyl (Me) | methyl (Me) | 270 +/- 43.8 |
| 14 | 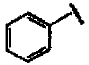 | benzyl (Bn) | 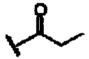 | 170 +/- 28.5 |
| 15 | 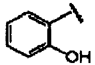 | H | 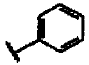 | 113 +/- 20.4 |
| 16 | 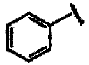 | H | 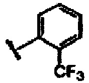 | 71 +/- 11 |
| 17 | 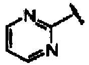 | ethyl (Et) | 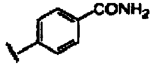 | 68 +/- 11 |
| 18 | 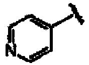 | benzyl (Bn) | 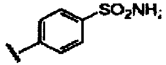 | 39 +/- 6.2 |
| 19 | 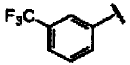 | H | 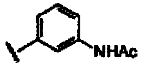 | 23 +/- 3.4 |
| 20 | 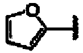 | H | 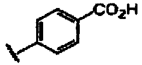 | 10 +/- 1.3 |
| 21 | 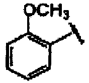 | H | 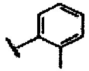 | 4.4 +/- 0.64 |
| 22 | 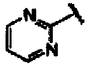 | H | 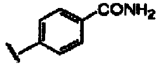 | 2.6 +/- 0.28 |
| 23 | 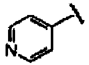 | H | 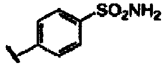 | 0.31 +/- 0.13 |

| | | | | |
|---|---|---|---|---|
| (*) values are reported as the concentration required to inhibit, by 50%, the binding of a fluorescently labeled mannosylated glycoconjugate to CD209 tetramer. | | | | |

Variations of each of the above compounds may additionally contain spacers, such as carbon chains, and in particular carbon chains containing between 2 and 50-carbon atoms, between the formula III molecule and the R₃ group.

In more specific embodiments, the compound may have the general chemical formula of formula (III), in which R₁, R₂, and R₃ may each be an alkyl group, an aryl group, a substituent with both aryl and alkyl components, or H.

In further specific embodiments, the identity of R₁ may be more determinative of the ability to bind to CD209 than the identity of R₂ or R₃. R₁ may be a piperazine derivative functionalized with an aliphatic substituent, but for a more strongly binding compound, R₁ may contain an aryl group, particularly a nitrogen heterocycle.

In still further specific embodiments, R₂ may be H or another small substituent, with a decrease in CD209 binding observed with larger substituents.

In another specific embodiment, R₃ may contain an aryl group with a polar substituent in order to improve CD209 binding.

In a more specific embodiment, both R₁ and R₃ may contain an aryl group for improved CD209 binding.

In still other embodiments, the compound may contain or be derived from a quinoxalinone scaffold of the general chemical formula: 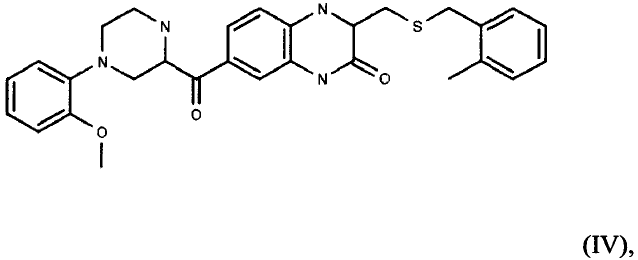 or a pyrazolone scaffold of the general chemical formula:

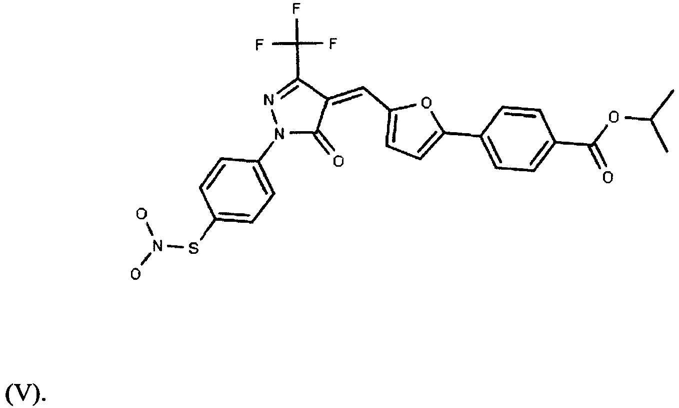

Further, described herein is a compound having the general chemical formula:

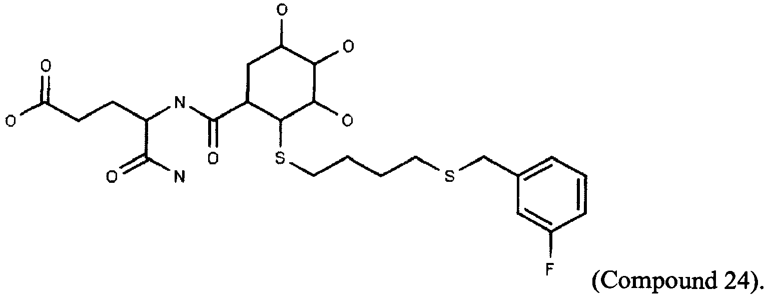

Furthermore, described herein is a compound having the general chemical formula:

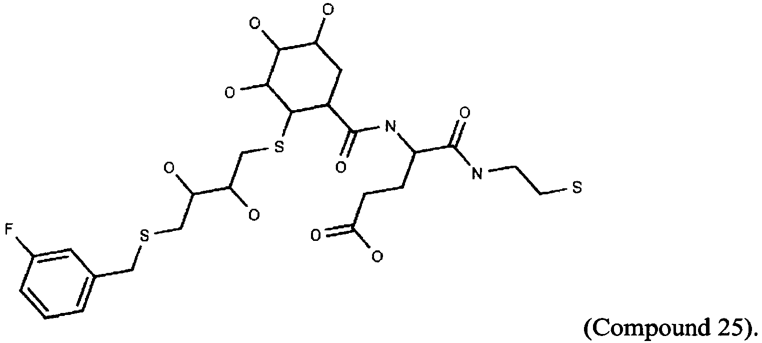

In still other embodiments, the compound may contain or may be conjugated to a carrier protein (e.g. Albumin) to form a glycoprotein ligand of CD209.

In still other embodiments, the compound may contain or be conjugated to a variety of scaffolds to form multivalent systems.

In another embodiment, the compound may include an artificial fucose mimic with the general chemical formula:

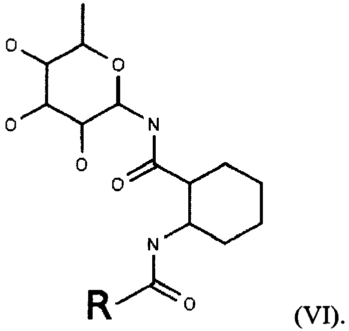

In more specific embodiments, the compound may have the general chemical formula of formula (VI), in which R may be an alkyl group, an aryl group, a substituent with both aryl and alkyl components, or H.

In further specific embodiments, the identity of R is determinative of the ability to bind to CD209.

In further specific embodiments, the *cis*-β- amino acid backbone of compound (VI) may determine the specificity and the ability of this compound to bind CD209.

Further, described herein is a compound having the general chemical formula:

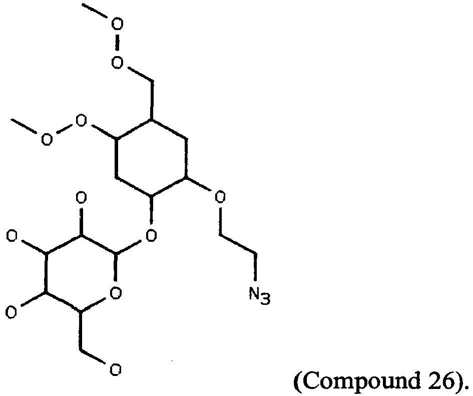

Also described herein is a compound having the general chemical formula:

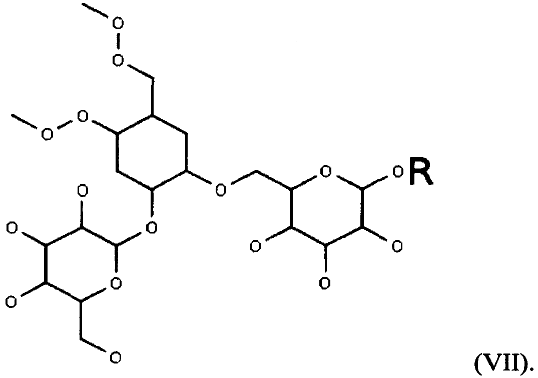

In more specific embodiments, the compound may have the general chemical formula of formula (VII), in which R may be an alkyl group, an aryl group, a substituent with both aryl and alkyl components, or H.

In further specific embodiments, the identity of R is determinative of the ability to bind to CD209.

In more specific embodiments, the compound (VII) may be conjugated to Bolton-type dendrons to form multivalent structures.

In another embodiment, the compound may contain glactoside or mannoside in a polymeric form

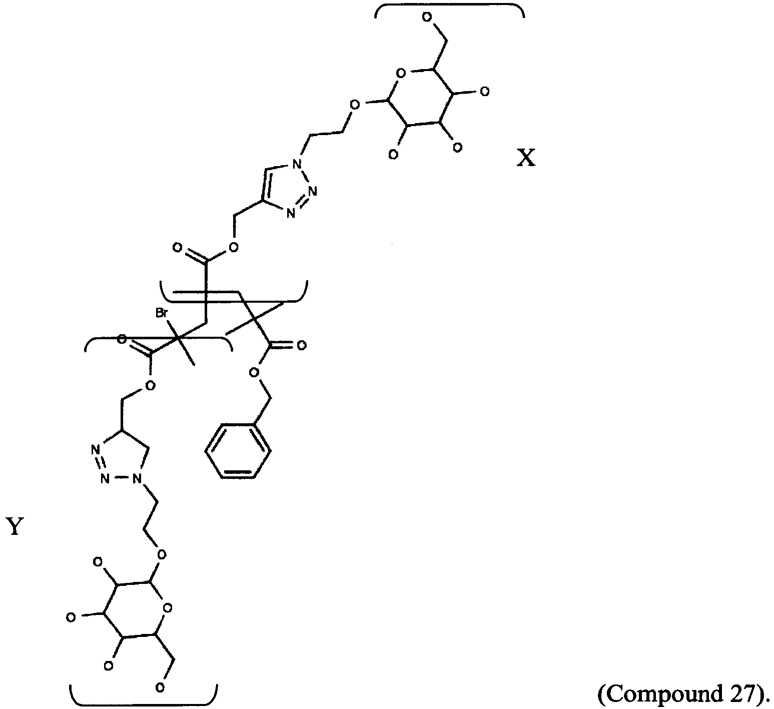

In more specific embodiments, compound 27 may have anywhere between 1 to hundreds of X or Y monomers to form a polymeric ligand of CD209.

In more specific embodiments, the number of X and Y monomers present in compound 27 determines its affinity for CD209.

CD209 plays a role in human immunodeficiency virus (HIV) infection, Ebola, Dengue fever, and Hepatitis C. Thus small molecules able to bind to CD209 and block binding of these pathogens to CD209 may also generally be used to prevent or treat fibrosing diseases.

Additional small molecules able to bind CD209 along with principles for designing additional such molecules and synthesis methods may be found in Borrak, M and Kiessling, L, Non-carbohydrate inhibitors of the lectin DC-SIGN, J. Am. Chem. Soc. 129: 12780-12785 (2007),Mangold, S et al., Quinoxalinone inhibitors of the lectin DC-SIGN, Chem. Sci. 3: 772-777 (2012), Sattin, S., A. Daghetti, M. Thépaut, A. Berzi, M. Sanchez-Navarro, G. Tabarani, J. Rojo, F. Fieschi, M. Clerici, and A. Bernardi. 2010. Inhibition of DC-SIGN-mediated HIV infection by a linear trimannoside mimic in a tetravalent presentation. ACS chemical biology 5: 301-12, Becer, C. R., M. I. Gibson, J. Geng, R. Ilyas, R. Wallis, D. a Mitchell, and D. M. Haddleton. 2010. High-affinity glycopolymer binding to human DC-SIGN and disruption of DC-SIGN interactions with HIV envelope glycoprotein. Journal of the American Chemical Society 132: 15130-2, Luczkowiak, J., S. Sattin, I. Sutkevičiutė, J. J. Reina, M. Sanchez-Navarro, M. Thépaut, L. Martinez-Prats, A. Daghetti, F. Fieschi, R. Delgado, A. Bernardi, and J. Rojo. 2011. Pseudosaccharide functionalized dendrimers as potent inhibitors of DC-SIGN dependent Ebola pseudotyped viral infection. Bioconjugate chemistry 22: 1354-65, Andreini, M., D. Doknic, I. Sutkeviciute, J. J. Reina, J. Duan, E. Chabrol, M. Thepaut, E. Moroni, F. Doro, L. Belvisi, J. Weiser, J. Rojo, F. Fieschi, and A. Bernardi. 2011. Second generation of fucose-based DC-SIGN ligands: affinity improvement and specificity versus Langerin. Organic & biomolecular chemistry 9: 5778-86,Prost, L. R., J. C. Grim, M. Tonelli, and L. L. Kiessling. 2012. Noncarbohydrate glycomimetics and glycoprotein surrogates as DC-SIGN antagonists and agonists. ACS chemical biology 7: 1603-8.

Anti-human CD209 antibodies may also be used to inhibit fibrocyte formation, profibrotic macrophages, and fibrosis. These antibodies include but are not limited to clone 9E9A8 anti-CD209 antibody (Biolegend, San Diego, CA), clone H-200 and 1B10 anti-CD209 antibody (Santa Cruz biotechnology, Dallas, TX), clone DCN46 (BD Biosciences, San Jose, CA), clone eB-h209 (eBioscience, San Diego, CA), and clone ERP5588 (Epitomics, Burlingame, CA). Antibodies may include monoclonal antibodies, polyclonal antibodies, antibody fragments, human antibodies, humanized antibodies, chimeric antibodies, bispecific antibodies, recombinant antibodies, IgG1 antobodies, and IgG2 antibodies. LL. Antibodies may include antibodies that bind to the same epitopes as clone 9E9A8, clone 1B10 clone H-200, clone DCN46, clone eB-h209, or clone ERP5588.

### Pharmaceutical Formulations

Pharmaceutical formulations may contain at least one or a combination of compounds or antibodies able to bind CD209 and inhibit formation of fibrocytes, inhibit profibrotic macrophages, or promote the formation of immunoregulatory macrophages from monocytes.

In specific embodiments, the formulation may be tailored for its intended use. For example, the formulation may be suitable for administration via oral administration, topical administration, injection or inhalation. An inhalable formulation may be suitable for use in a nebulizer. Topical formulations may include any suitable cream, ointment, emollient, gel, foam, or transdermal patch as a carrier. An oral formulation may include pills, tablets, capsules, liquids, syrups, and food or drink supplements, which may include extended release formulations.

In each formulation, the compound or compounds may be contained in therapeutically effective amounts, which may vary depending on the intended mode of administration and the ease of access to the blood stream or fibrosis site using the selected mode of administration. In one specific embodiment, a therapeutically effective amount may be that sufficient to result in a concentration of at least 3 pM at the site of a fibrotic lesion or in the vicinity of a monocyte likely to inappropriately form a fibrocyte or profibrotic macrophage. In other specific embodiments, the therapeutically effective amount may result in a concentration of at least 300 pM or at least 2 nM in the same types of locations. In another embodiment, a therapeutically effective amount may be that sufficient to result in a concentration of at least 3 pM, at least 300 pM, at least 2 nM, or another concentration able to inhibit fibrocyte or profibrotic macrophage formation when the concentration is measured in blood serum.

Formulations of these compounds may also include a pharmaceutically acceptable carrier, in particular a carrier suitable for the intended mode of administration, or salts, buffers, or preservatives. In particular embodiments containing biological molecules, such an antibodies or other proteins, the pharmaceutically acceptable carrier may be tailored to allow the biological molecule to retain an active conformation and to avoid degradation.

In specific embodiments, where Ca²⁺ or another cation, such as a divalent cation contributes to binding of CD209 by the compound, the formulation may contain a sufficient amount of Ca²⁺ or other cation, such as a divalent cation to allow or enhance binding.

In still additional specific embodiments, the pharmaceutical formulation may contain stabilizers to prevent or decrease degradation of the compound or to otherwise render the formulation stable at temperatures able to prevent or decrease degradation of the compound. Sulfur (S)-containing compounds may, in particular, be prone to thermal degradation and may thus need stabilizers or storage at a low temperature.

Pharmaceutical formulations may include other pharmaceutically effective materials. For example, formulations may include Serum Amyloid P (SAP).

Pharmaceutical formulations may also be prepared for antibodies, which may be administered at a concentration of 10 micrograms/ml or less, at a concentration of 1 micrograms/ml or less, at a concentration of 0.1 micrograms/ml or less, at a concentration of between 0.1 micrograms/ml and 10 micrograms/ml, or at a concentration of between 0.1 micrograms/ml and 10 micrograms/ml.

### Preventing or Treating Fibrosing Diseases

Any of the above compounds and pharmaceutical formulations may be used to inhibit the formation of fibrocytes or profibrotic macrophages from monocytes or to treat or prevent a fibrosing disease in a target location by administering the compound or formulation to the target location in a sufficient dose for a sufficient amount of time.

In some fibrosing diseases fibrocytes or profibrotic macrophages may not represent an end-stage of fibrosis. For example, in asthma, fibrocytes further differentiate into myofibroblasts, which persist in thickened airway walls.

Generally, compounds and pharmaceutical formulations may treat or prevent fibrosis resulting from conditions including but not limited to rheumatoid arthritis, lupus, pathogenic fibrosis, fibrosing disease, fibrotic lesions such as those formed after Schistosoma japonicum infection, radiation damage, autoimmune diseases, Lyme disease, chemotherapy induced fibrosis, HIV or infection-induced focal sclerosis, failed back syndrome due to spinal surgery scarring, abdominal adhesion post-surgery scarring, fibrocystic formations, fibrosis after spinal injury, surgery-induced fibrosis, mucosal fibrosis, peritoneal fibrosis caused by dialysis, Adalimumab-associated pulmonary fibrosis, and nephrogenic fibrosing dermopathy.

Specifically, in the liver, compounds and pharmaceutical formulations may treat or prevent fibrosis resulting from conditions including but not limited to alcohol, drug, or chemically induced cirrhosis, ischemia-reperfusion injury after hepatic transplant, necrotizing hepatitis, hepatitis B, hepatitis C, primary biliary cirrhosis, and primary sclerosing cholangitis.

Relating to the kidney, compounds and pharmaceutical formulations may treat or prevent fibrosis resulting from conditions including but not limited to proliferative and sclerosing glomerulonephritis, nephrogenic fibrosing dermopathy, diabetic nephropathy, renal tubulointerstitial fibrosis, and focal segmental glomerulosclerosis.

Relating to the lung, compounds and pharmaceutical formulations may treat or prevent fibrosis resulting from conditions including but not limited to pulmonary interstitial fibrosis, sarcoidosis, pulmonary fibrosis, idiopathic pulmonary fibrosis, asthma, chronic obstructive pulmonary disease, diffuse alveolar damage disease, pulmonary hypertension, neonatal bronchopulmonary dysplasia, chronic asthma, and emphysema. There are several sub-names or synonyms for pulmonary fibrosis including, but not limited to, cryptogenic fibrosing alveolitis, diffuse interstitial fibrosis, idiopathic interstitial pneumonitis, Hamman-Rich syndrome, silicosis, asbestosis, berylliosis, coal worker's pneumoconiosis, coal miner's disease, miner's asthma, anthracosis, and anthracosilicosis.

Relating to the heart or pericardium, compounds and pharmaceutical formulations may treat or prevent fibrosis resulting from conditions including but not limited to myocardial fibrosis, atherosclerosis, coronary artery restenosis, congestive cardiomyopathy, heart failure, and other post-ischemic conditions.

Relating to the eye, compounds and pharmaceutical formulations may treat or prevent fibrosis resulting from conditions including but not limited to exophthalmos of Grave's disease, proliferative vitreoretinopathy, anterior capsule cataract, corneal fibrosis, corneal scarring due to surgery, trabeculectomy-induced fibrosis, progressive subretinal fibrosis, multifocal granulomatous chorioretinitis, fibrosis due to wide angle glaucoma trabeculotomy, and other eye fibrosis.

Relating to the brain, they may treat or prevent fibrosis resulting from conditions including but not limited to glial scar tissue.

Relating to the skin, compounds and pharmaceutical formulations may treat or prevent fibrosis resulting from conditions including but not limited to Depuytren's contracture, scleroderma, keloid scarring, psoriasis, hypertrophic scarring due to burns, atherosclerosis, restenosis, and psuedoscleroderma caused by spinal cord injury.

Relating to the mouth or esophagus, compounds and pharmaceutical formulations may treat or prevent fibrosis resulting from conditions including but not limited to periodontal disease scarring, gingival hypertrophy secondary to drugs, and congenital esophageal stenosis.

Relating to the pancreas, compounds and pharmaceutical formulations may treat or prevent fibrosis resulting from conditions including but not limited to pancreatic fibrosis, stromal remodeling pancreatitis, and stromal fibrosis.

Relating to the gastrointestinal tract, compounds and pharmaceutical formulations may treat or prevent fibrosis resulting from conditions including but not limited to collagenous colitis, villous atrophy, crypt hyperplasia, polyp formation, fibrosis of Crohn's disease, and healing gastric ulcer.

Relating to the breast, compounds and pharmaceutical formulations may treat or prevent fibrosis resulting from conditions including but not limited to fibrocystic disease and desmoplastic reaction to breast cancer.

Relating to the bone marrow, compounds and pharmaceutical formulations may treat or prevent fibrosis resulting from conditions including but not limited to fibrosis in myelodysplasia and neoplastic diseases.

Relating to the bone, compounds and pharmaceutical formulations may treat or prevent fibrosis resulting from conditions including but not limited to rheumatoid pannus formation.

Relating to the genitourinary system, compounds and pharmaceutical formulations may treat or prevent fibrosis resulting from conditions including but not limited to endometriosis, uterine fibroids, ovarian fibroids, and Peyronie's disease.

Relating to radiation induced damage, compounds and pharmaceutical formulations may treat or prevent fibrosis related to, but not limited to, treatment of head and neck cancer, ovarian cancer, prostate cancer, lung cancer, gastrointestinal cancer, colon cancer, and breast cancer.

Fibrosing diseases may be treated in any patients, including mammals and humans.

One or more target locations may be present in the patient.

### Methods of Detecting CD209 Binding Compounds and Antibodies

The present disclosure further includes methods of screening for compounds and antibodies able to reduce or suppress formation of fibrocytes or profibrotic macrophages from monocytes. Such methods involve first screening compound and antibodies to determine if they bind to CD209, then screening any binding compounds to detect whether they also to reduce or suppress formation of fibrocytes or profibrotic macrophages from monocytes. Binding screening may be conduced using high-throughput techniques.

### EXAMPLES

The following examples provide further details regarding certain aspects of the disclosure and are not intended to describe the complete invention.

### Example 1: Identification of glycosylation as differentiating factor between SAP and CRP

To understand why SAP inhibits fibrocyte formation, while CRP does not, a mutational study was carried out to identify amino acids that contribute the ability of recombinant human SAP to inhibit fibrocyte formation. First, amino acids that are different between SAP and CRP were identified, then mapped to the crystal structure of human SAP to identify protein-surface amino acids that were different from CRP. Although some surface amino acids partially contribute to the ability of SAP to inhibit fibrocyte formation, no single amino acid or combination of amino acids were identified that could abolish this ability of SAP.

Given that SAP is glycosylated and CRP is not, glycosylation was identified as a possible cause for the different effects of these two proteins. The effects of unglycosylated SAP could not be tested because the glycosylation could not be removed completely without impeding the secretion of the protein (data not shown). Instead, the terminal sialic acids from the SAP molecule were removed enzymatically by neuraminidase to prevent glycosylation. In addition, CRP was glycosylated and the ability of glycosylated CRP to influence fibrocyte and profibrotic macrophage formation was tested.

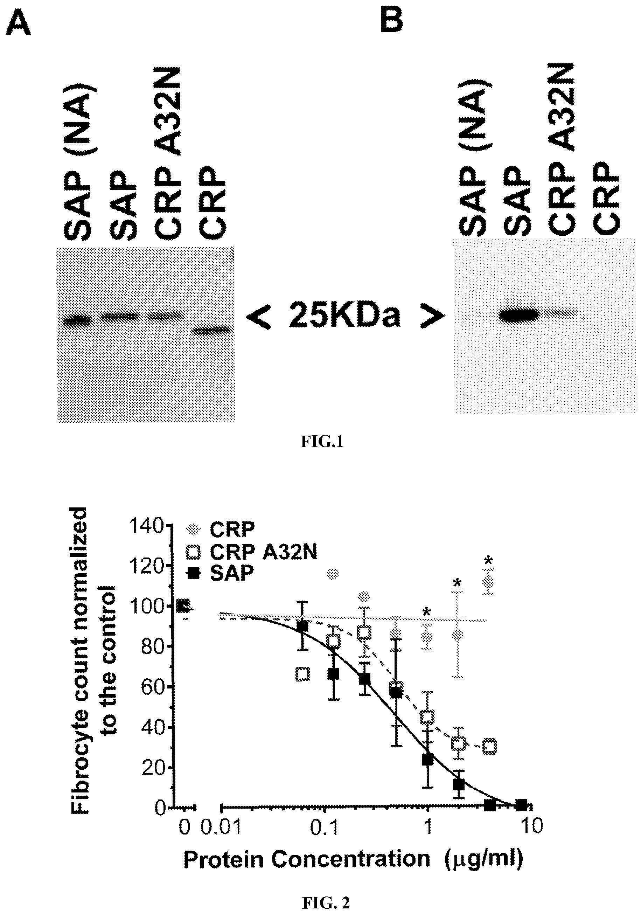

CRP was glycosylated by mutating its 32^{nd} amino acid from alanine to asparagine, the glycosylated amino acid on SAP. The crystal structure of CRP revealed that this 32^{nd} amino acid is located on the surface of CRP, where it may be glycosylated. A SDS-PAGE gel showed an apparent molecular mass of 25 KDa for modified CRP (CRP A32N), similar to SAP, and dissimilar to wild type CRP, which has an apparent molecular mass of 23 KDa (FIGURE 1A). A western blot of SAP and the modified CRP (CRP A32N), stained with Sambucus Nigra lectin, which detects α (2,6)-glycosidic linkages of sialic acid, showed the presence of sialic acid on SAP and CRP A32N (FIGURE 1B). Neuraminidase-treated SAP (SAP (NA)) and CRP were not stained with the Sambucus Nigra lectin (FIGURE 1B). These results confirm that SAP contains sialic acids, and that CRP does not contain sialic acids, and indicate that neuraminidase treatment of SAP removes the sialic acids, and that CRP A32N contains sialic acid.

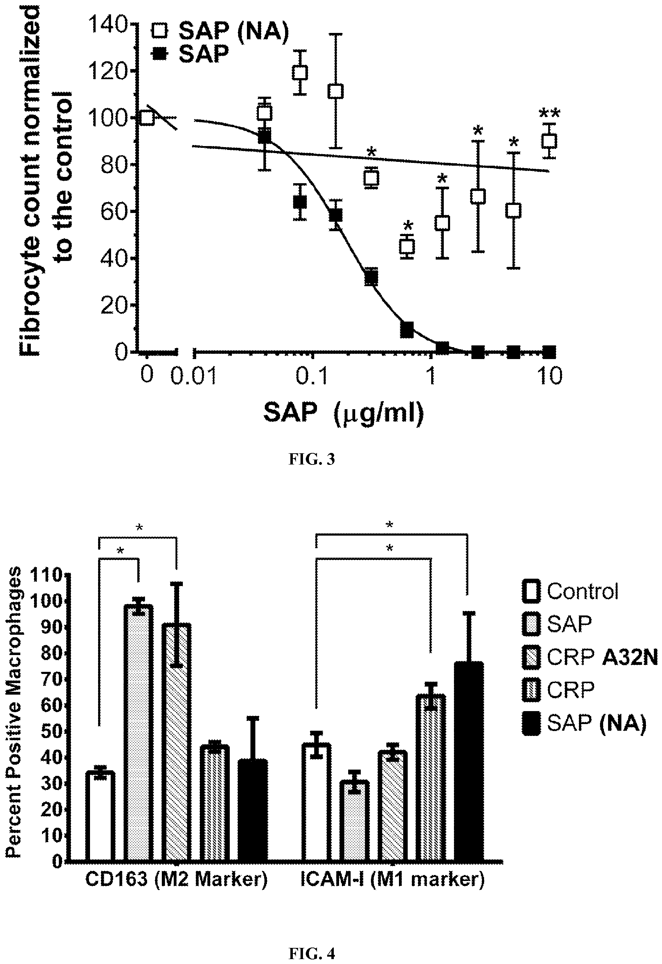

The modified-glycosylated CRP (CRP A32N) was able to able to inhibit fibrocyte formation, although not as well as SAP (FIGURE 2). Specifically, at 0.25 microgram/ml and above for SAP, and 1 microgram/ml and above for modified-glycosylated CRP, the proteins significantly inhibited fibrocyte formation, with p < 0.05 (t-test). Enzymatically desialylated SAP (SAP NA) which lacks the terminal sialic acids (FIGURE 1B) had reduced inhibitory effect on fibrocyte differentiation (FIGURE 3). When tested on macrophages, 3 microgram/ml of SAP and CRP A32N both increased the expression of the M2 marker CD163, while 3 microgram/ml of CRP and SAP NA did not (FIGURE 4). Conversely, CRP and SAP NA both increased the expression of the M1 marker ICAM-1, while SAP and CRP A32N did not cause such an increase (FIGURE 4). These results suggest that glycosylation plays a role in SAP's ability to inhibit fibrocyte formation and influence macrophage polarization, resulting in the formation of CD163-positive immuno-regulatory M2 macrophages, but the SAP protein sequence also remains partially responsible for the inhibitory effect of SAP on fibrocyte and profibrotic macrophage formation.

### Example 2: Identification of CD209 as glycosylation receptor

CD209 was identified as a candidate glycosylation receptor for SAP and modified-glycosylated CRP because it is present on the surface of monocytes. CD209 normally binds the α(2,6)-glycosidic linkages of sialic acid on immunoglobulins to increase Interleukin 33 production and therefore decrease macrophage activation in presence of proinflammatory signals. α(2,6)-glycosidic linkages of sialic acid are also found on SAP.

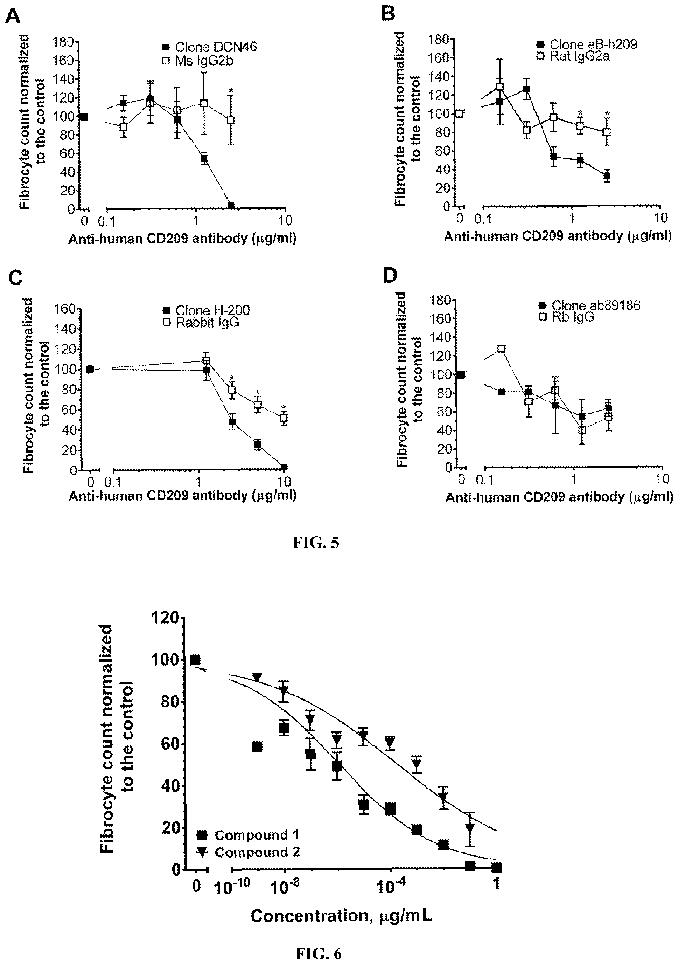

To determine if CD209 could regulate fibrocyte formation, human peripheral blood mononuclear cells (PBMCs) were treated with different anti-CD209 antibodies. Three anti-CD209 antibodies (clones DCN46, eB-h209, and H-200), when added to human PBMCs, inhibited fibrocyte formation (FIGURE 5A-C). Anti-CD209 antibody clone ab89186 had no significant effect on human fibrocyte differentiation (FIGURE 5D). More specifically, at 2.5 microgram/ml the anti-CD209 antibody clone DCN46 significantly inhibited fibrocyte formation, while the mouse IgG2b had only a modest effect on fibrocyte formation (FIGURE 5A). At 1.25 microgram/ml and above, the anti-CD209 antibody clone eB-h209 significantly inhibited fibrocyte formation, whereas the rat IgG2a had only a modest effect on fibrocyte formation (FIGURE 5B). At 2.5 microgram/ml and above, the anti-CD209 antibody clone H-200 significantly inhibited fibrocyte formation, whereas the rabbit IgG had only a modest effect on fibrocyte formation (FIGURE 5C). These results indicate that some but not all anti-CD209 antibodies can inhibit fibrocyte differentiation.

### Example 3: Small molecule CD209 ligands inhibit fibrocyte formation

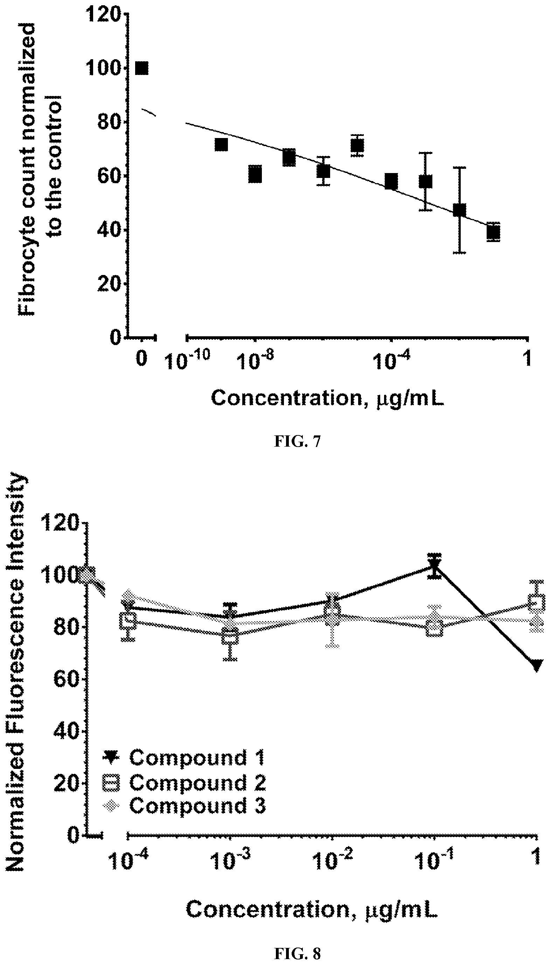

Small molecules known to interfere with CD209 binding to mannose were tested for their ability to inhibit fibrocyte formation. Compound 1 inhibited fibrocyte formation from human PBMCs with an IC₅₀ of 3.1 pM (FIGURE 6). Compound 2 inhibited fibrocyte formation from human PBMCs as well with an IC₅₀ of 330 pM (FIGURE 6). Compound 3 also significantly inhibited fibrocyte formation with an IC₅₀ of 2.2 nM (FIGURE 7). Compound 3 significantly inhibited fibrocyte formation at all tested concentrations with p < 0.05 (t-test).

To ascertain if the inhibitory effect was genuine, Alamar Blue was used to estimate cell viability in PBMCs. None of the three small molecules had any significant effect on cell viability (FIGURE 8).

Thus, compounds able to inhibit mannose binding to CD209 clearly also have the capacity to inhibit fibrocyte formation from monocytes, often at very low concentrations. Other CD209 ligands are expected to have the same effect.

### Example 4: A small molecule CD209 ligands inhibit fibrosis in mice

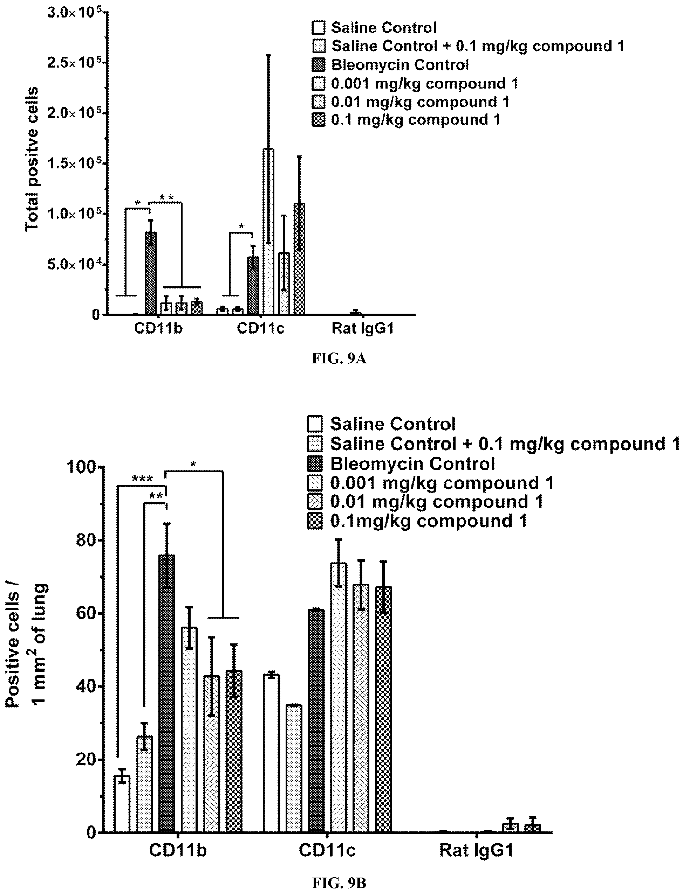

To determine the effects of compound 1 in treatment of a fibrosing disease, pulmonary fibrosis was selected as a model. Pulmonary fibrosis was induced in C57/BL6 mice by oropharyngeal aspiration of bleomycin into their lungs. 24 hour after bleomycin administration, mice received daily intraperitoneal injections of compound 1. At day 21 mice were euthanized and the bronchoalveolar lavage fluid (BAL) and the lungs were harvested. At 0.1 mg/kg, 0.01mg/kg, and 0.001 mg/kg, compound 1 significantly decreased CD11b positive inflammatory macrophages in the BAL as compared to the bleomycin control (FIGURE 9A). CD11c positive macrophages were increased significantly in the BAL following bleomycin treatment but were not altered significantly with daily injections of compound 1 (FIGURE 9A). At 0.1 mg/kg and 0.01mg/kg, compound 1 significantly decreased the number of CD11b positive inflammatory macrophages in the lung sections after weakly-adherent cells were removed by BAL (FIGURE 9B). Together, these results indicate that compound 1 decreases the number of inflammatory macrophages in the lungs.

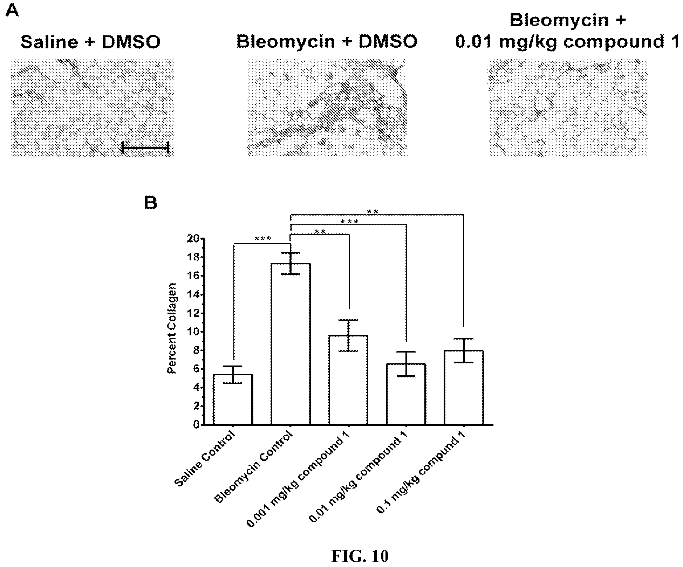

Cryosections of the mouse lungs were stained with picrosirius red to estimate the collagen content of the lungs. These collagen measurements are summarized in FIGURE 9. Specifically, collagen content in the lungs of mice that had received bleomycin alone was significantly higher compared to that of mice administered only 0.9% saline (FIGURE 10). In contrast, mice administered bleomycin and compound 1 (at 0.1 mg/kg, 0.01mg/kg, and 0.001 mg/kg) showed significantly less collagen in the lungs than mice that received bleomycin, indicating that compound 1 helps prevent the development of fibrosis in the lungs and the accompanying accumulation of collagen.

### Example 5: Methods

The following materials and methods were used in connection with Examples 1, 2, 3, and 4.

### Reagents, antibodies and small molecules

Mouse anti-human CD209 antibody clone H200 was obtained from Santa Cruz Biotechnology (Dallas, TX). Mouse anti-human CD163 and ICAM-1 antibodies were obtained from Biolegend Inc (San Diego, CA). Mouse anti-human CD209 antibody clone DCN46 was obtained from BD Biosciences (San Jose, CA). Rat anti-human CD209 antibody clone eB-h209 was obtained from eBioscience (San Jose, CA). Rabbit anti-human CD209 antibody clone ab89186 was obtained from Abcam (Cambridge, MA). Compound 1 (Cat#5931866) was purchased from ChemBridge (San Diego, CA). Compound 2 (Cat#545-1519) and compound 3 (Cat#4545-1530) were purchased from ChemDiv (San Diego, CA).

### SDS-PAGE gels and western blotting

Proteins were diluted in 20 mM sodium phosphate, pH 7.4 to a concentration of 40 microgram/ml. Samples were then mixed with Laemmli's sample buffer, containing 20 mM dithiothreitol, and heated to 100°C for 5 minutes. Samples (10 microliters) were loaded onto 4-20% Tris/glycine polyacrylamide gels (Bio-Rad, Hercules, CA). Gels were stained with silver stain as described previously by Pilling, D. et al., Inhibition of fibrocyte differentiation by serum amyloid p, Journal of Immunology 171:5537-5546 (2003). For Western blotting, proteins from gels were transferred to polyvinylidene difluoride membranes (Immobilon P; Millipore, Bedford, MA) in Tris/glycine/SDS buffer containing 20% methanol following the manufacturer's protocol. Filters were blocked for 30 minutes at room temperature in Carbo-Free Blocking Solution (Vector Laboratories, Burlingame, CA). Membranes were then incubated in PBS containing 10 microgram/ml of Biotinylated Sambucus Nigra Lectin (Vector Laboratories) for 30 minutes at room temperature to detect α (2,6)-glycosidic linkages of sialic acid following the manufacturer's protocol. A Vectastain N Elite ABC peroxidase kit (Vector Laboratories) was used to detect the biotinnylated lectin following manufacturer's protocol. Chemiluminescence (Clarity Western ECL substrate, Bio-Rad) was used following the manufacturer's protocol to visualize the peroxidase, using a Bio-Rad ChemiDoc XRS+ System.

### PRMC isolation, and cell viability, fibrocyte formation, and macrophage polarization assays

Human blood was collected into heparin tubes (BD Bioscience, San Jose, CA) from adult volunteers who gave written consent and with specific approval from the Texas A&M University human subjects Institutional Review Board. PBMCs were then isolated as previously described in Pilling, D. et al., Improved serum-free culture conditions for differentiation of human and murine fibrocytes, Journal of Immunology Methods 351:62-70 (2009). Alamar Blue (Life Technologies, Grand Island, NY) was used to assess cell viability following the manufacturer's protocol. Fibrocyte differentiation assays in the presence of different compounds were performed in serum-free medium as described in Pilling, D. et al., Improved serum-free culture conditions for differentiation of human and murine fibrocytes, Journal of Immunology Methods 351:62-70 (2009). Macrophage polarization assays to detect formation of proinflammatory macrophages with an M1 phenotype were carried out in serum-free medium, in which case monocytes were left to differentiate to macrophages for 5 days and then the medium was replaced with fresh medium containing 3 microgram/ml of the tested protein.

### Animal models of pulmonary fibrosis and bronchoalveolar lavage fluid

4-week-old 20 g C57/BL6 mice (Jackson, Bar Harbor, ME.) were treated with an oropharyngeal aspiration of 50 microliters of 0.9% saline or 3 U/kg bleomycin (Calbiochem, EMD Millipore, Billerica, MA.) in 50 microliters of 0.9% saline as described previously by Lakatos, HF. et al., Oropharyngeal aspiration of a silica suspension produces a superior model of silicosis in the mouse when compared to intratracheal instillation, Experimental Lung Reasearch 32:181-199 (2006. The successful aspiration of bleomycin into the lungs was confirmed by listening for the crackling noise heard after the aspiration. Starting 24 hours following bleomycin aspiration (day 1), mice were treated daily with intraperitoneal injections of 50 microliters of PBS containing 2 microliters of 1 mg/ml of compound 1 (ChemBridge Corporation, San Diego, CA) (0.1 mg/kg) dissolved in DMSO (SigmaAldrich), 2 microliters of 0.1 mg/ml of compound 1 (0.01 mg/kg) dissolved in DMSO, 2 microliters of 0.01 mg/ml of compound 1 (0.001 mg/kg) dissolved in DMSO, or an equal volume of PBS containing 2 microliters of DMSO. All animals were used in accordance with rules published by the National Institutes of Health and with a protocol approved by the Texas A&M University Institutional Animal Care and Use Committee. Mice were weighed daily, and euthanized using CO₂ following NIH guidelines at day 21 after bleomycin aspiration. No injections were done on day 21. After euthanasia, the lungs were perfused with 300 microliters of phosphate buffered saline (PBS), pH 7.4, three times to collect cells by bronchoalveolar lavage (BAL) as described previously by Lakatos, HF. et al., Oropharyngeal aspiration of a silica suspension produces a superior model of silicosis in the mouse when compared to intratracheal instillation, Experimental Lung Reasearch 32:181-199 (2006). The BAL cells from the first BAL collection were collected by centrifugation at 500×g for 10 minutes, and the supernatants were transferred to Eppendorf tubes. The supernatants were flash frozen with liquid nitrogen, and stored at -20° C. until further use. The BAL pellets were resuspended in the secondary and tertiary BAL fluid and the combined cells were collected by centrifugation at 500×g for 5 minutes. The cells were resuspended in 500 microliters of 4% (w/v) BSA-PBS and counted with a hemocytometer. 100 microliters of diluted cells were then aliquoted into cytospin funnels and were then spun onto glass slides (Superfrost plus white slides, VWR) at 400×g for 5 minutes using a cytospin centrifuge (Shandon, Cheshire, England). These cells were then air-dried, and stained for CD11b (Biolegend) or CD11c (MBL International, Woburn, MA) as described below.

### Immunohistochemical studies

Macrophage staining with anti-CD163 and anti-ICAM-1 antibodies was carried out as described in Pilling, D. et al., Identification of markers that distinguish monocyte-derived fibrocytes from monocytes, macrophages, and fibroblasts, PloS one 10:e7475 (2009). Both primary antibodies were used at a concentration of 5 microgram/ml.

After collection of BAL fluid, mouse lungs were inflated with prewarmed OCT (VWR, Radnor, PA) and then embedded in OCT, frozen on dry ice, and then stored at -80°C. 6-micron cryosections of mouse lung were mounted on Superfrost Plus microscope slides (VWR). Sections were fixed in acetone for 20 min at room temperature. Nonspecific binding was then blocked by incubation in 4% BSA (SigmaAldrich) in PBS for 60 min. Slides were then incubated with 5 microgram/ml primary antibodies in PBS containing 4% BSA for 60 min. Mouse lung sections were stained for CD11b (Biolegend, San Diego, CA) and CD11c (MBL International). Isotype-matched mouse antibody (Biolegend) was used as negative control. Slides were then washed in three changes of PBS over 30 minutes and incubated for 30 minutes with 2 microgram/ml biotinylated donkey anti-rat IgG (Jackson ImmunoResearch Laboratories, Inc., West Grove, PA) in PBS. After washing, the biotinylated antibodies were detected by streptavidin-alkaline phosphatase (Vector Laboratories) following the manufacturer's protocol. For picrosirius red staining, 6-µm cryosections of mouse lung were mounted on Superfrost Plus microscope slides (VWR) and fixed in acetone for 20 minutes. Sections were then left to dry for 10 minutes. Afterwards, lung section were hydrated in water for 10 minutes and then incubated for 5 minutes with 0.2% phosphomolybdic acid. Cryosection were rinsed 3x in water and incubated with 1 mg/ml Sirius Red (Polysciences, Warrington, PA) in water saturated with picric acid for 10 minutes. The lung section were washed 2x, 5 minutes each with PBS. Lung sections were then rinsed in acidified water (5 ml acetic acid in 1 L of water) twice and washed in water for 5 minutes. Lung sections were air dried for 10 minutes, mounted, and imaged.

### CRP mutation

The cDNA for human CRP was mutated at residue 32 using a QuickChange II Site-Directed Mutagenesis Kit (Agilent, Santa Clara, CA) and the primer 5' AAGGCGCGCCATGGAGAAGCTGTTG 3'(SEQ. ID. NO. 1). CRP cDNA was inserted into a pCMV6-AC-His (ORigene, Rockville, MD) vector and then expressed in HEK293 cells (Life Technologies, Grand Island, NY) as previously described for the expression SAP in Crawford, JR, et al., Fc gamma RI mediates serum amyloid P inhibition of fibrocyte differentiation, J. Leukoc. Biol. 92: 699-711 (2012). CRP was purified using immobilized p-aminophenyl phosphoryl choline beads as described previously in Roper, MG, et al., Extraction of C-reactive protein from serum on a microfluidic chip. Analytica Chimica Acta 569: 195-202 (2006).

### Statistical analysis

Prism (GraphPad Software, San Diego, CA) was used for all statistical analysis.

## Claims

1. A CD209-binding compound for use in a method of suppressing fibrocyte or profibrotic macrophage formation, wherein the compound is Compound 1 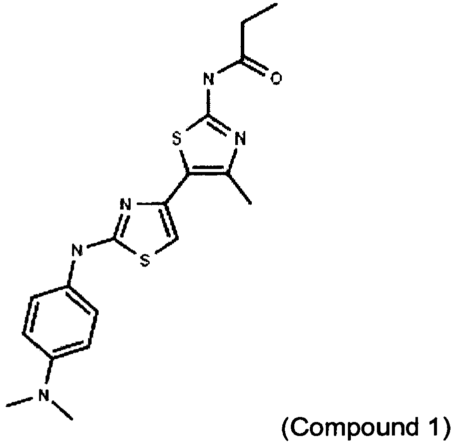 and is administered to monocytes in a target location in a lung of a mammal that has fibrosis or a fibrosing disease affecting the lung.

2. The compound for use of Claim 1, wherein the compound is administered by a mode selected from the group consisting of: topically, orally, by injection, by intravenous injection, by inhalation, continuous release by depot or pump, or any combinations thereof.

3. The compound for use of Claim 1, wherein the compound is administered in an amount and for a time until the target location in the mammal contains the compound in an amount of at least 3 pM.

## Patentansprüche

1. CD209-bindende Verbindung zur Verwendung in einem Verfahren zur Unterdrückung der Bildung von Fibrozyten oder von profibrotischen Makrophagen, wobei die Verbindung Verbindung 1 ist, 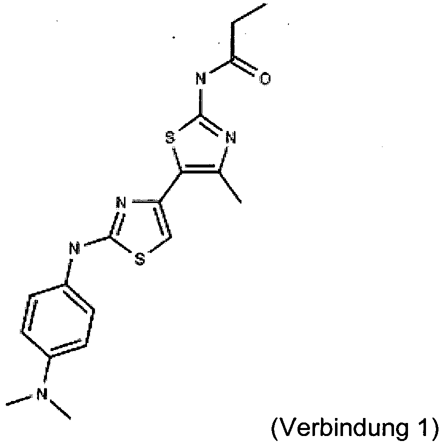 und and Monozyten an einem Zielort in der Lunge eines Säugetiers verabreicht wird, das eine Fibrose oder eine fibrosierende Erkrankung aufweist, welche die Lunge betrifft.

2. Verbindung zur Verwendung nach Anspruch 1, wobei die Verbindung durch einen Modus verabreicht wird, ausgewählt aus der Gruppe bestehend aus: topisch, oral, durch, Injektion, durch intravenöse Injektion, durch Inhalation, durch kontinuierliche Freisetzung mittels Depot oder Pumpe oder Kombinationen davon.

3. Verbindung zur Verwendung nach Anspruch 1, worin die Verbindung in einer Menge und für eine Zeitspanne verabreicht wird, bis der Zielort im Säugetier die Verbindung in einer Menge von mindestens 3 pM enthält.

## Revendications

1. Composé de fixation à CD209 destiné à être utilisé dans un procédé de suppression de la formation de fibrocytes ou macrophages profibrotiques, dans lequel le composé est le Composé 1 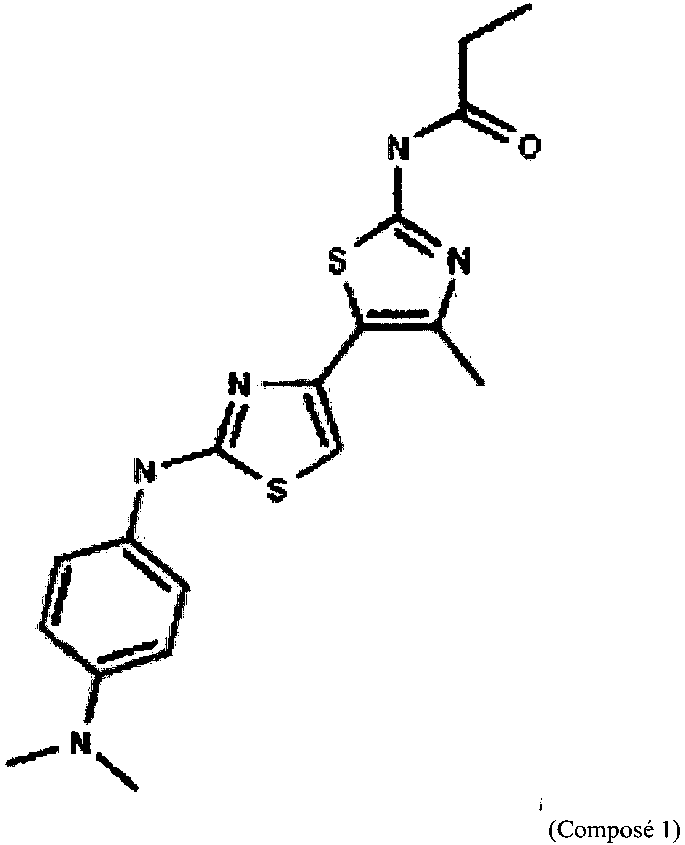 et est administré à des monocytes dans un emplacement cible dans un poumon d'un mammifère qui a une fibrose ou une maladie fibrosante affectant le poumon.

2. Composé destiné à être utilisé selon la revendication 1, dans lequel le composé est administré par un mode sélectionné parmi le groupe constitué de : voie topique, orale, par injection, par injection intraveineuse, par inhalation, libération continue par dépôt ou pompe, ou toutes combinaisons de ceux-ci.

3. Composé destiné à être utilisé selon la revendication 1, dans lequel le composé est administré en une quantité et pendant une durée jusqu'à ce que l'emplacement cible chez le mammifère contienne le composé en une quantité d'au moins 3 pM.
